(19) Europäisches Patentamt
European Patent Office
Office européen des brevets

(11) Veröffentlichungsnummer: **0 035 972**
**B1**

(12) # EUROPÄISCHE PATENTSCHRIFT

(45) Veröffentlichungstag der Patentschrift: **19.10.83**

(51) Int. Cl.³: **C 07 C 127/19**, A 01 N 47/30, C 07 D 211/16, C 07 D 295/20

(21) Anmeldenummer: **81810074.5**

(22) Anmeldetag: **04.03.81**

(54) **Cyanoalkyl-Phenylharnstoffe mit selektiver herbizider Wirkung, deren Herstellung und sie enthaltende Mittel.**

(30) Priorität: **10.03.80 CH 1860/80**

(43) Veröffentlichungstag der Anmeldung: **16.09.81 Patentblatt 81/37**

(45) Bekanntmachung des Hinweises auf die Patenterteilung: **19.10.83 Patentblatt 83/42**

(84) Benannte Vertragsstaaten: **BE CH DE FR GB IT LI NL**

(56) Entgegenhaltungen: **US-A-3 125 432**

(73) Patentinhaber: **CIBA-GEIGY AG, Patentabteilung Postfach, CH-4002 Basel (CH)**

(72) Erfinder: **Pissiotas, Georg, Dr., Breslauerstrasse 8, D-7850 Lörrach (DE)**
Erfinder: **Dürr, Dieter, Dr., Brändelistalweg 16, CH-4103 Bottmingen (CH)**
Erfinder: **Rohr, Otto, Dr., Kilbertweg 19, CH-4106 Therwil (CH)**
Erfinder: **Lukaszczyk, Alfons, Bruderholzweg 38, CH-4053 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

Cyanoalkyl-Phenylharnstoffe mit selektiver herbizider Wirkung, deren Herstellung und sie enthaltende Mittel

Die vorliegende Erfindung betrifft neue Cyanoalkyl-phenylharnstoffe mit herbizider Wirkung, deren Herstellung, sie enthaltende Mittel, sowie deren Verwendung zur selektiven Unkrautbekämpfung in verschiedenen Kulturen wie z. B. Baumwolle, Mais, insbesondere aber Getreide und Raps. Ferner können diese Wirkstoffe mit ähnlichem Erfolg in Zuckerrübenkulturen eingesetzt werden.

Herbizid wirkende Phenylharnstoffe sind längst bekannt und eingeführt (z. B. US-A-3 125 432). Durch ihre Verbreitung konnten Kulturen wie Getreide einerseits weitgehend von Unkraut befreit werden, anderseits ist es zu einer Proliferation von Unkräutern gekommen, die gegenüber solchen Herbiziden resistenter sind.

Die erfindungsgemäßen neuen Cyanoalkyl-phenylharnstoffe entsprechen der allgemeinen Formel I

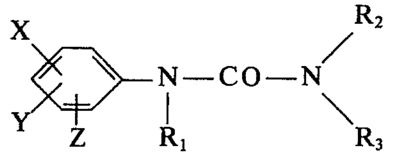

(I)

worin

X Wasserstoff, Halogen, Trifluormethyl, Methyl oder Methoxy,

Y eine Cyanoalkylgruppe 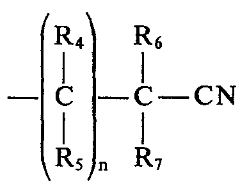

Z Wasserstoff oder Halogen,
n die Zahl 0, 1 oder 2,
$R_1$ Wasserstoff oder $C_1-C_6$-Alkyl,
$R_2$ Wasserstoff, $C_1-C_6$-Alkyl, $C_1-C_6$-Alkoxy, $C_2-C_6$-Alkenyl oder $C_3-C_6$-Alkinyl,
$R_3$ Wasserstoff, $C_1-C_6$-Alkyl, $C_2-C_6$-Alkenyl oder
$R_2$ und $R_3$ zusammen mit dem Stickstoff, an das sie gebunden sind, einen 5–7gliedrigen Heterocyclus bilden, der auch ein Sauerstoff- oder Schwefelatom oder eine Iminogruppe als Ringglied enthalten kann,
$R_4$, $R_5$, $R_6$ Wasserstoff, $C_1-C_6$-Alkyl oder $C_1-C_6$-Halogenalkyl,
$R_7$ Wasserstoff, $C_1-C_6$-Alkyl, Aralkyl, insbesondere Benzyl, Phenyl oder $C_1-C_6$-Alkoxy oder
$R_6$ und $R_7$ zusammen mit dem sie tragenden Kohlenstoffatom einen $C_3-C_7$-Cycloalkylring bilden können, bedeuten.

Unter Halogen ist in der Regel Fluor, Chlor oder Brom zu verstehen, wobei Chlor oder Brom bevorzugte Halogene sind.

Unter Alkyl oder dem Alkylteil eines anderen Substituenten wie Alkoxy, Halogenalkyl, Aralkyl ist je nach Anzahl der Kohlenstoffatome zu verstehen: Methyl, Äthyl, n-Propyl, i-Propyl, Butyl, Pentyl, Hexyl sowie die entsprechenden isomeren Reste wie z. B. sek-Butyl, i-Butyl, n-Pentyl, neo-Pentyl.

Sinngemäß sind demnach Beispiele für Alkoxy: Methoxy, Äthoxy, i-Propyloxy; für Halogenalkyl: Chlormethyl, Difluormethyl, Trifluormethyl, 2,2,2-Trifluoräthyl, Perfluoräthyl und für Arakyl: Benzyl, 2-Phenyläthyl, 1-Phenyläthyl.

Unter Alkenyl versteht man im allgemeinen: Vinyl, Allyl, Methallyl, 1-Butenyl, 2-Butenyl, 3-Butenyl sowie die isomeren Pentenyl und Hexenyl, insbesondere aber Allyl.

Alkinyl steht beispielsweise für Propargyl, 2-Butinyl, 3-Butinyl, sowie die isomeren Pentinyl und Hexinyl, insbesondere aber für Propargyl.

Definitionsgemäße Cycloalkylreste sind: Cyclopropyl, Cyclobutyl, Cyclopentyl, Cyclohexyl, Cycloheptyl.

Beispiele für 5 bis 7gliedrige Stickstoffheterocyclen sind: Pyrrolidin, Pyrazolidin, Imidazolidin, Oxazolidin, Thiazolidin, Piperidin, Piperimidin, Piperazin, Morpholin, Thiomorpholin, Perhydroazepin.

Die Verbindungen der Formel I weisen ausgesprochen selektiv-herbizide Eigenschaften im allgemeinen auf und erweisen sich als besonders vorteilhaft zum Bekämpfen von Unkräutern in Kulturen von Nutzpflanzen wie Baumwolle, Mais und insbesondere in Getreide. Einzelne Verbindungen wirken selektiv in Raps. Ferner können auch Zuckerrübenanpflanzungen mit diesen Wirkstoffen zur Unkrautbekämpfung behandelt werden.

Bevorzugt sind diejenigen Verbindungen der Formel I, in denen

a) der Cyanoalkylsubstituent Y die para- oder meta-Position des Phenylrings besetzt,
b) n für Null oder eins steht,
c) $R_2$ für Wasserstoff, Methyl oder Methoxy und $R_3$ für Methyl stehen und
d) $R_1$ Wasserstoff,
e) Z Wasserstoff und
f) X Wasserstoff, Halogen oder Trifluormethyl bedeuten.

Eine weitere Bevorzugung ist in denjenigen Verbindungen zu sehen, in denen

aa) der Cyanoalkylsubstituent Y die para-Position des Phenylrings besetzt und n Null bedeutet, und
bb) der Cyano-alkylsubstituent Y die meta-Position des Phenylrings besetzt und n eins bedeutet.

Durch weitere Kombination der Merkmale der bevorzugten Gruppen ergeben sich folgende ganz besonders interessante Gruppen:

aaa) Verbindungen, in denen die Substituenten Y in der para-Stellung und X in der meta-Stellung des Phenylkernes stehen, n Null ist, $R_1$ und Z Wasserstoff, $R_2$ Wasserstoff, Methyl oder Methoxy, $R_3$ Methyl und X Wasserstoff, Chlor oder Trifluormethyl bedeuten und
bbb) Verbindungen, in denen der Substituent Y in der meta-Stellung des Phenylkerns steht, n eins ist, $R_1$, X und Z Wasserstoff, $R_2$ Wasserstoff, Methyl oder Methoxy und $R_3$ Methyl bedeuten.

Als bevorzugte Einzelverbindungen sind zu nennen:

β-[4-(3,3-Dimethylureido)-3-chlorphenyl]-propionitril,
β-[4-(3-Methoxy-3-methylureido)-3-chlorphenyl]-propionitril,
β-[3-(3-Methoxy-3-methylureido)-phenyl]-propionitril,
β-[3-(3,3-Dimethylureido)-phenyl]-propionitril,
α-[4-(3-Methoxy-3-methylureido)-3-chlorphenyl)-acetonitril,
α-[4-(3,3-Dimethylureido)-3-chlorphenyl]-acetonitril,
α-[4-(3,3-Dimethylureido)-3-trifluormethylphenyl]-acetonitril,
β-[4-(3,3-Dimethylureido)-3-methylphenyl]-α-methyl-propionitril.

Bei genügend großer Aufwandmenge ist jedoch auch totalherbizide Wirkung vorhanden. Die Anwendung kann sowohl im Vorauflauf – wie im Nachlaufverfahren vorgenommen werden. Dabei können die Aufwandmengen in weiten Grenzen schwanken, z. B. zwischen 0,1 bis 10 kg Wirkstoff pro Hektar, vorzugsweise werden jedoch 0,5 bis 5 kg Wirkstoff pro Hektar eingesetzt.

Erfindungsgemäße Mittel enthalten außer einem Wirkstoff der Formel I einen geeigneten Träger und/oder andere Zuschlagstoffe. Geeignete Träger und Zuschlagstoffe können fest oder flüssig sein und entsprechen den in der Formulierungstechnik üblichen Stoffen, wie z. B. natürlichen oder regenerierten mineralischen Stoffen, Lösungs-, Verdünnungs-, Dispergier-, Emulgier-, Netz-, Haft-, Verdickungs-, Binde- und/oder Düngemitteln.

Die herbiziden Mittel mit einem Gehalt an einem Wirkstoff der Formel I können in der Form von Stäubemittel, Emulsionskonzentraten, Granulaten, Dispersionen oder auch als Lösungen oder Aufschlämmungen, hergestellt nach üblichen Formulierungsmethoden, vorliegen.

Die Herstellung der Verbindungen der Formel I erfolgt nach an sich bekannten Methoden.

Gemäß einem ersten Verfahren werden die Cyanoalkyl-phenylharnstoffe der Formel I hergestellt, indem man in einem gegenüber den Reaktionsteilnehmern inerten organischen Lösungsmittel ein entsprechend substituiertes Anilin der Formel II

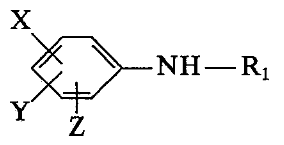

(II)

in Gegenwart eines säurebindenden Mittels mit einem Carbamoylchlorid der Formel III

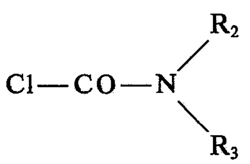

(III)

worin X, Y, Z, $R_1$, $R_2$ und $R_3$ die unter Formel I gegebene Bedeutung haben, umsetzt.

Gemäß einem weiteren Verfahren werden die Cyanoalkyl-phenylharnstoffe der Formel I, in denen $R_1$

Wasserstoff bedeutet, hergestellt, indem man ein entsprechend substituiertes Isocyanat der Formel IV

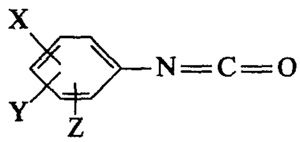

(IV)

mit einem Amin der Formel V

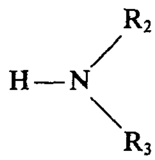

(V)

worin X, Y, Z, $R_2$ und $R_3$ die unter Formel I gegebene Bedeutung haben, umsetzt.

Die als Ausgangsmaterialien benötigten Isocyanate IV erhält man durch Umsetzen entsprechend substituierter Aniline der Formel VI

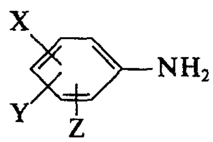

(VI)

mit Phosgen, worin X, Y und Z die unter Formel I gegebene Bedeutung haben.

Diese Reaktionen werden in den Reaktionspartnern gegenüber inerten organischen Lösungsmitteln vorgenommen, wie Ketonen, z. B. Aceton, Äthylmethylketon, Cyclohexanon; Estern, z. B. Äthylacetat; Dimethylformamid; Acetonitril; Äthern wie Diäthyläther, Tetrahydrofuran, Dioxan etc. Es ist vorteilhaft, wenn die Lösungsmittel mit Wasser mischbar sind, da sich das Reaktionsprodukt durch Wasserzugabe ausfällen läßt. Die Reaktionstemperaturen liegen zwischen 0°C und 150°C, praktisch zwischen Raumtemperatur und dem Siedepunkt des Reaktionsgemisches. Im allgemeinen wird unter Normaldruck gearbeitet, größere Mengen können mit Vorteil auch in Druckgefäßen hergestellt werden.

Die Verbindungen der Formel I sind für Warmblüter wenig giftig, ihre Handhabung bedarf keiner vorsorglichen Maßnahmen. Sie sind in den üblichen organischen Lösungsmitteln relativ gut und in Wasser schlecht löslich. Sie können durch Zugeben von Wasser zur Reaktionslösung leicht ausgefällt werden. Ihre Formulierung als flüssige herbizide Mittel gelingt mit Hilfe üblicher Lösungsvermittler und/oder Dispergiermittel.

Die neuen Wirkstoffe der Formel I sind stabile Verbindungen, welche in üblichen organischen Lösungsmitteln, wie Alkoholen, Äthern, Ketonen, Dimethylformamid, Dimethylsulfoxid etc. löslich sind.

Die Herstellung erfindungsgemäßer Mittel erfolgt in an sich bekannter Weise durch inniges Vermischen und Vermahlen von Wirkstoffen der allgemeinen Formel I mit geeigneten Trägerstoffen und/oder Verteilungsmitteln, gegebenenfalls unter Zusatz von gegenüber den Wirkstoffen inerten Antischaum-, Netz-, Dispersions- und/oder Lösungsmitteln. Die Wirkstoffe können in den folgenden Aufarbeitungsformen vorliegen und angewendet werden:

Feste Aufarbeitungsformen:
Stäubemittel, Streumittel, Granulate, Umhüllungsgranulate, Imprägniergranulate und Homogengranulate;

In Wasser dispergierbare Wirkstoffkonzentrate:
Spritzpulver (wettable powder), Pasten, Emulsionen, Emulsionskonzentrate, Suspensionskonzentrate (flowable);

Flüssige Aufarbeitungsformen:
Lösungen.

Die Wirkstoffkonzentrationen betragen in den erfindungsgemäßen Mitteln 1 bis 80 Gewichtsprozent und können gegebenenfalls bei der Anwendung auch in geringen Konzentrationen wie etwa 0,05 bis 1% vorliegen.

Den erfindungsgemäßen Mitteln lassen sich andere biozide Wirkstoffe oder Mittel beimischen. So können die neuen Mittel außer den genannten Verbindungen der allgemeinen Formel I z. B. Insektizide, Fungizide, Bakterizide, Fungistatika, Bakteriostatika, Nematozide oder weitere Herbizide zur Verbreiterung des Wirkungsspektrums enthalten.

Die folgenden Beispiele 1 und 2 sollen die Herstellung der erfindungsgemäßen Phenylharnstoffe der Formel I näher erläutern. Weitere in analoger Weise hergestellte Verbindungen sind in den sich den Beispielen 1 und 2 anschließenden Tabellen 1 und 2 aufgeführt. Die Temperaturen sind in Celsiusgraden angegeben, Teile und Prozentangaben beziehen sich auf das Gewicht. Druckangaben sind in Torr (1 Torr = 1,33 millibar) angegeben. In weiteren Beispielen wird die Aufbereitung der Wirkstoffe zu technisch verwendbaren Verabreichungsformen sowie Versuche zur Demonstration der herbiziden Wirkung beschrieben.

## Herstellungsbeispiele

### Beispiel 1

β-[3-(3,3-Dimethylureido)-phenyl]-propionitril

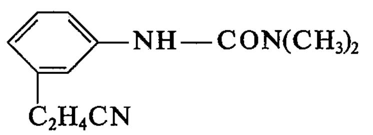

(Verbindung 101)

a) 115 g β-(3-Nitrophenyl)-propionitril werden in eine siedende Mischung von 150 g Eisenspänen, 5 ml konzentrierter Salzsäure, 100 ml Wasser, 300 ml Alkohol und 150 ml Toluol eingetragen. Man kocht die Mischung 4 Stunden am Rückfluß und läßt dann erkalten. Dann gibt man 1 Liter ca. 0,5 normaler Natronlauge dazu und filtriert. Der Filterrückstand wird mit Toluol gewaschen. Die Toluollösungen werden abgetrennt, eingedampft und der Rückstand destilliert. Man erhält so 50 g β-(3-Aminophenyl)-propionitril vom Siedepunkt 132°/0,08 Torr.

b) 50 g β-(3-Aminophenyl)-propionitril werden in 100 ml Essigester gelöst und bei −20° in eine Lösung von 40 g Phosgen in 300 ml Toluol zutropfen gelassen. Man rührt diese Lösung dann 8 Stunden bei Raumtemperatur und kocht sie schließlich noch eine Stunde am Rückfluß. Dann wird das Lösungsmittel abdestilliert. Der Rückstand, bestehend aus öligem β-(3-Isocyanatophenyl)-propionitril, welches bei 126°/0,5 Torr siedet, wird unter Rühren zu einer Mischung von 25 g einer 40%igen wäßrigen Lösung von Dimethylamin in 200 ml Acetonitril zutropfen gelassen. Schließlich gibt man Eiswasser zur Reaktionslösung, wodurch der Harnstoff ausfällt. Man filtriert und kristallisiert das erhaltene β-[3-(3,3-Dimethylureido)-phenyl]-propionitril aus verdünntem Äthanol um. Ausbeute 33 g, Schmelzpunkt 159–161°.

### Beispiel 2

α-[4-(3-Methoxy-3-methylureido)-3-chlorphenyl]-acetonitril

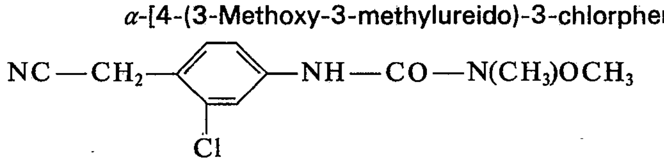

(Verbindung 23)

a) 128 g α-(3-Chlor-4-nitrophenyl)-acetonitril werden in eine siedende Mischung von 150 g Eisenspänen, 5 ml konzentrierter Salzsäure, 100 ml Wasser, 300 ml Alkohol und 150 ml Toluol eingetragen. Man kocht die Mischung 4 Stunden am Rückfluß und läßt dann erkalten. Dann gibt man 1 Liter 0,5 normaler Natronlauge dazu und filtriert. Der Filterrückstand wird mit Toluol gewaschen. Die Toluollösungen werden abgetrennt, eingedampft und der Rückstand destilliert. Man erhält so 57 g α-(4-Amino-3-chlorphenyl)-acetonitril vom Schmelzpunkt 77°.

b) 57 g α-(4-Amino-3-chlorphenyl)-acetonitril werden in 100 ml Essigester gelöst und bei −20° in eine Lösung von 40 g Phosgen in 300 ml Toluol zutropfen gelassen. Man rührt diese Lösung dann 8 Stunden bei Raumtemperatur und kocht sie schließlich noch eine Stunde am Rückfluß. Dann wird das Lösungsmittel abdestilliert. Der Rückstand, bestehend aus öligem α-(4-Isocyanato-3-chlorphenyl)-acetonitril, wird unter Rühren zu einer Lösung von 13,5 g Methoxymethylamin in 200 ml Acetonitril zutropfen gelassen. Schließlich gibt man Eiswasser zur Reaktionslösung, wodurch der Harnstoff ausfällt. Man filtriert und kristallisiert das erhaltene α-[4-(3-Methoxy-3-methylureido)-3-chlorphenyl)-acetonitril aus verdünntem Äthanol um. Ausbeute 38 g, Schmelzpunkt 104°.

In analoger Weise zu diesen Beispielen werden folgende Verbindungen hergestellt:

Tabelle 1

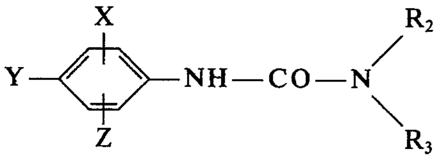

| Nr. | Y | X | Z | $NR_2R_3$ | Phys. Daten |
|---|---|---|---|---|---|
| 1 | $NC-CH_2-$ | H | H | $N(CH_3)OCH_3$ | Smp. 85–86° |
| 2 | $NC-CH(CH_3)-$ | H | H | $N(CH_3)OCH_3$ | Smp. 103–104° |
| 3 | $NC-C(CH_3)_2-$ | H | H | $N(CH_3)OCH_3$ | Smp. 94–95° |
| 4 | 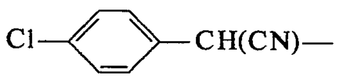 | H | H | $N(CH_3)OCH_3$ | Smp. 96–97° |
| 5 | 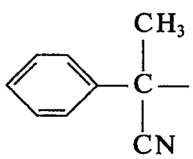 | H | H | $NHCH_3$ | Smp. 168–169° |
| 6 | 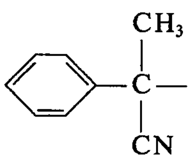 | H | H | $N(CH_3)OCH_3$ | $n_D^{40}$ 1.5723 |
| 7 | $NC-CH(C_2H_5)-$ | H | H | $N(CH_3)_2$ | Smp. 162–164° |
| 8 | $NC-CH(C_2H_5)-$ | H | H | $N(CH_3)OCH_3$ | Smp. 67–68° |
| 9 | $NC-C_2H_4-$ | H | H | $N(CH_3)_2$ | Smp. 91–93° |
| 10 | $NC-C_2H_4-$ | H | H | $N(CH_3)OCH_3$ | Smp. 63–65° |
| 11 | $NC-CH(C_4H_9$ sek$)-$ | H | H | $N(CH_3)OCH_3$ | viskoses Öl |
| 12 | $NC-C_2H_4-$ | 3-Cl | H | $N(CH_3)_2$ | Smp. 128° |
| 13 | $NC-C_2H_4-$ | 3-Cl | H | $N(CH_3)OCH_3$ | Smp. 88–90° |
| 14 | $NC-CH(CH_3)-CH_2-$ | H | H | $N(CH_3)_2$ | Smp. 103–104° |
| 15 | $NC-CH(CH_3)-CH_2-$ | H | H | $N(CH_3)OCH_3$ | Smp. 99–101° |
| 16 | $NC-C_2H_4-$ | 3-$CF_3$ | | $N(CH_3)_2$ | Smp. 158–160° |
| 17 | $NC-C_2H_4-$ | 3-$CF_3$ | | $N(CH_3)OCH_3$ | Smp. 121–123° |
| 18 | 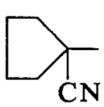 | H | H | $N(CH_3)_2$ | Smp. 128–130° |
| 19 | 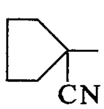 | H | H | $N(CH_3)OCH_3$ | Smp. 104–105° |

Fortsetzung

| Nr. | Y | X | Z | $NR_2R_3$ | Phys. Daten |
|---|---|---|---|---|---|
| 20 | $NC-CH(CH_3)-$ | 3-Cl | H | $N(CH_3)_2$ | |
| 21 | $NC-CH(CH_3)-$ | 3-Cl | H | $N(CH_3)OCH_3$ | |
| 22 | $NC-CH_2-$ | 3-Cl | H | $N(CH_3)_2$ | Smp. 176–178° |
| 23 | $NC-CH_2-$ | 3-Cl | H | $N(CH_3)OCH_3$ | Smp. 104° |
| 24 | 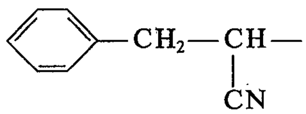 | H | H | $N(CH_3)_2$ | Smp. 155° |
| 25 | 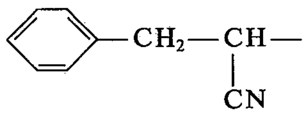 | H | H | $N(CH_3)OCH_3$ | Smp. 130° |
| 26 | 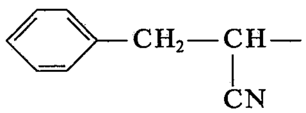 | 3-Cl | H | $N(CH_3)_2$ | |
| 27 | 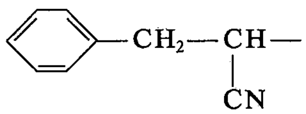 | 3-Cl | H | $N(CH_3)OCH_3$ | |
| 28 | $NC-CH_2-$ | $3-CF_3$ | H | $NHC_2H_5$ | Smp. 112° |
| 29 | $NC-C_2H_4-$ | $3-CH_3$ | H | $N(CH_3)_2$ | Smp. 96–97° |
| 30 | $NC-C_2H_4-$ | $3-CH_3$ | H | $N(CH_3)OCH_3$ | Smp. 30° |
| 31 | $NC-CHCl-CH_2-$ | 3-Cl | H | $N(CH_3)_2$ | |
| 32 | $NC-CHCl-CH_2-$ | 3-Cl | H | $N(CH_3)OCH_3$ | |
| 33 | $NC-CHCl-CH_2-$ | H | H | $NHCH_3$ | |
| 34 | $NC-CH(CH_3)-CH_2-$ | $3-CH_3$ | H | $N(CH_3)_2$ | Smp. 83–85° |
| 35 | $NC-CH(CH_3)-CH_2-$ | $3-CH_3$ | H | $N(CH_3)OCH_3$ | |
| 36 | $NC-CH(CH_3)-CH_2-$ | H | H | $NHCH_3$ | |
| 37 | $NC-CH(C_6H_5)-CH_2-$ | H | H | $N(CH_3)_2$ | Smp. 134–138° |
| 38 | $NC-CH_2-$ | 3-Cl | H | $NHCH_3$ | Smp. 162–164° |
| 39 | $NC-CH_2-$ | $3-CF_3$ | H | $N(CH_3)_2$ | Smp. 155° |
| 40 | $NC-CH_2-$ | $3-CF_3$ | H | $N(CH_3)OCH_3$ | Smp. 86° |
| 41 | $NC-C_2H_4-$ | 3-Cl | H | $N(CH_3)C_2H_5$ | Smp. 93–95° |
| 42 | $NC-(CH_2)_3-$ | H | H | $N(CH_3)_2$ | Smp. 79–82° |
| 43 | $NC-(CH_2)_3-$ | H | H | $N(CH_3)OCH_3$ | Smp. 72° |

Fortsetzung

| Nr. | Y | X | Z | $NR_2R_3$ | Phys. Daten |
|---|---|---|---|---|---|
| 44 | $NC-CH(CH_3)-CH(CH_3)-$ | H | H | $N(CH_3)_2$ | Öl |
| 45 | $NC-CH(CH_3)-CH(CH_3)-$ | H | H | $N(CH_3)OCH_3$ | Öl |
| 46 | $NC-CH_2-CH(CH_3)-$ | H | H | $N(CH_3)OCH_3$ | Smp. 30° |
| 47 | $NC-CH_2-CH(CH_3)-$ | H | H | $N(CH_3)_2$ | Smp. 89–90° |
| 48 | $NC-CH_2-CH(C_2H_5)-$ | H | H | $N(CH_3)_2$ | $n_D^{27} = 1.5658$ |
| 49 | $NC-CH_2-CH(C_2H_5)-$ | H | H | $N(CH_3)OCH_3$ | $n_D^{25} = 1.5526$ |
| 50 | $NC-CH(CH_3)-CH_2-$ | H | H | $N(CH_3)_2$ | Smp. 173° |
| 51 | $NC-CH_2-$ | $3-CF_3$ | H | $N(CH_3)-n-C_4H_9$ | Smp. 50° |
| 52 | $NC-CH_2-$ | $3-CF_3$ | H | 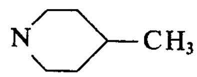 | Smp. 157° |
| 53 | $NC-CH_2-$ | $3-CF_3$ | H | $NHCH_3$ | Smp. 126° |
| 54 | $NC-CH_2-$ | $3-CF_3$ | H | $NH-n-C_4H_9$ | Smp. 128–130° |
| 55 | $NC-CH_2-$ | 3-Cl | H | $N(CH_2-CH=CH_2)_2$ | Smp. 87° |
| 56 | $NC-CH_2-$ | 3-Cl | H | $NH-CH_2-CH=CH_2$ | Smp. 103–105° |
| 57 | $NC-CH_2-$ | 3-Cl | H | 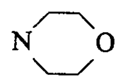 | Smp. 136° |
| 58 | $NC-CH_2-$ | 3-Cl | H | 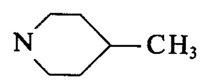 | Smp. 117–120° |
| 59 | $NC-CH_2-$ | 3-Cl | H | $N(CH_3)-n-C_4H_9$ | Smp. 96–99° |
| 60 | $NC-CH_2-$ | 3-Cl | H | $N(CH_3)-CH-CH_3$<br>\|<br>$C\equiv CH$ | Smp. 99° |

Tabelle 2

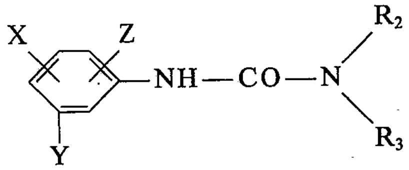

| Nr. | Y | X | Z | $NR_2R_3$ | Phys. Daten |
|---|---|---|---|---|---|
| 101 | $NC-C_2H_4-$ | H | H | $N(CH_3)_2$ | Smp. 159–161° |
| 102 | $NC-C_2H_4-$ | H | H | $N(CH_3)OCH_3$ | Smp. 72– 72° |
| 103 | $NC-C_2H_4-$ | 4-Br | H | $N(CH_3)_2$ | Smp. 147–149° |
| 104 | $NC-C_2H_4-$ | 4-Br | H | $N(CH_3)OCH_3$ | Smp. 98– 99° |
| 105 | $NC-C_2H_4-$ | 4-Cl | H | $N(CH_3)OCH_3$ | Smp. 103–105° |
| 106 | $NC-CH(CH_3)-CH_2-$ | $4-CH_3$ | H | $N(CH_3)_2$ | |
| 107 | $NC-CH(CH_3)-CH_2-$ | $4-CH_3$ | H | $N(CH_3)OCH_3$ | |
| 108 | $NC-C_2H_4-$ | $4-OCH_3$ | H | $N(CH_3)_2$ | |
| 109 | $NC-C_2H_4-$ | $4-OCH_3$ | H | $N(CH_3)OCH_3$ | |
| 110 | $NC-CH(CH_3)-CH_2-$ | 4-Cl | H | $N(CH_3)_2$ | Smp. 187–189° |
| 111 | $NC-CH(CH_3)-CH_2-$ | 4-Cl | H | $N(CH_3)OCH_3$ | Smp. 82– 84° |
| 112 | $NC-C_2H_4-$ | H | H | $N(CH_3)_2$ | |
| 113 | $NC-C_2H_4-$ | H | H | $N(CH_3)OCH_3$ | |
| 114 | $NC-C_2H_4-$ | 2-Cl | 4-Cl | $N(CH_3)OCH_3$ | Smp. 80° |
| 115 | $NC-C_2H_4-$ | $4-CH_2$ | H | $N(CH_3)_2$ | Smp. 178–179° |
| 116 | $NC-C_2H_4-$ | $4-CH_3$ | H | $N(CH_2)OCH_3$ | Smp. 87– 90° |
| 117 | $NC-C_2H_4-$ | $4-CH_3$ | H | $NHCH_3$ | Smp. 162–164° |
| 118 | $NC-C_2H_4-$ | 4-Cl | H | $N(CH_3)_2$ | Smp. 163–164° |
| 119 | $NC-CH(C_6H_5)-CH_2-$ | H | H | $N(CH_3)_2$ | Smp. 144–146° |
| 120 | $NC-CH_2-$ | H | H | $N(CH_3)OCH_3$ | $n_D^{25}$ = 1,5513 |
| 121 | $NC-CH_2-$ | H | H | $N(CH_3)_2$ | Smp. 115° |

## Formulierungsbeispiele

Das Aufbereiten der Verbindungen der Formel I zu für die Landwirtschaft brauchbaren Anwendungsformen kann beispielsweise gemäß den folgenden Vorschriften erfolgen:

### Beispiel 3

### Spritzpulver

Zur Herstellung eines a) 70%igen und b) 10%igen Spritzpulvers werden folgende Bestandteile verwendet:

a) 70 Teile $\beta$-[m-(3,3-Dimethylureido)-phenyl]-propionitril (Verbindung Nr. 101),
5 Teile Natriumdibutylnaphthylsulfonat,
3 Teile Naphthalinsulfonsäuren-Phenolsulfonsäuren-Formaldehyd-Kondensat 3 : 2 : 1,
10 Teile Kaolin,
12 Teile Champagne-Kreide,

b) 10 Teile $\alpha$-[p-(3-Methoxy-3-methylureido)-phenyl]-acetonitril (Verbindung Nr. 1),
3 Teile Gemisch der Natriumsalze von gesättigten Fettalkoholsulfaten,
5 Teile Naphthalinsulfonsäuren-Formaldehyd-Kondensat,
82 Teile Kaolin.

Der angegebene Wirkstoff wird auf die entsprechenden Trägerstoffe (Kaolin und Kreide) aufgezogen und anschließend vermischt und vermahlen mit den übrigen Bestandteilen. Man erhält Spritzpulver von vorzüglicher Vernetzbarkeit und Schwebefähigkeit. Aus solchen Spritzpulvern können durch Verdünnen mit Wasser Suspensionen von 0,1–80% Wirkstoff erhalten werden, die sich zur Unkrautbekämpfung in Pflanzenkulturen eignen.

Beispiel 4

Paste

Zur Herstellung einer 45%igen Paste werden folgende Stoffe verwendet:

45 Teile $\alpha$-[p-(3-Methoxy-3-methylureido)-phenyl]-isobutyronitril (Verbindung Nr. 3),
5 Teile Natriumaluminiumsilikat,
14 Teile Cetylpolyglykoläther mit 8 Mol Äthylenoxid,
1 Teil Oleylpolyglykoläther mit 5 Mol Äthylenoxid,
2 Teile Spindelöl,
10 Teile Polyäthylenglykol (mittleres Molekulargewicht 400 g/Mol),
23 Teile Wasser.

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

Beispiel 5

Emulsionskonzentrat

Zur Herstellung eines 25%igen Emulsionskonzentrates werden

25 Teile $\beta$-[4-Brom-(3,3-dimethylureido)-phenyl]-propionitril (Verbindung Nr. 103),
10 Teile einer Mischung von Nonylphenolpolyoxyäthylen oder Calciumdodecylbenzolsulfonat,
10 Teile Cyclohexanon,
55 Teile Xylol

miteinander vermischt. Dieses Konzentrat kann mit Wasser zu Emulsionen auf geeignete Konzentrationen von z. B. 0,1% verdünnt werden. Solche Emulsionen eignen sich zur Bekämpfung von Unkräutern in Kulturpflanzungen.

Beispiel 6

Suspensionskonzentrat

Zur Herstellung eines 45%igen Suspensionskonzentrates werden folgende Stoffe verwendet:

45 Teile $\alpha$-[p-(3-Methylureido)-phenyl]-$\alpha$-phenylpropionitril (Verbindung Nr. 5),
5 Teile Äthylenglykol,
3 Teile Octylphenoxypolyäthylenglykol mit 9–10 Mol Äthylenoxyd pro Mol Octylphenol),
3 Teile von einem Gemisch aromatischer Sulfonsulfosäuren, kondensiert mit Formaldehyd als Ammoniumsalz,
1 Teil Siliconöl in Form einer 75%igen Emulsion,

| | |
|---|---|
| 0,1 Teil | einer Mischung von 1-(3-Chlorallyl)-3,5,7-triazo-azonium-adamantan-chlorid mit Natriumcarbonat, Chloridwert mind. 11,5%, |
| 0,2 Teile | eines biopolymeren Verdickers mit max. 100 Keimen pro Gramm, |
| 42,7 Teile | Wasser. |

Die Aktivsubstanz wird mit den Zuschlagstoffen in dazu geeigneten Geräten innig vermischt und vermahlen. Man erhält ein Konzentrat aus dem sich durch Verdünnen mit Wasser Suspensionen jeder gewünschten Konzentration herstellen lassen.

## Biologische Beispiele

Zum Nachweis der Brauchbarkeit als Herbizide (pre- und post-emergent) dienen folgende Testmethoden:

### Beispiel 7

### Pre-emergente Herbizid-Wirkung

Im Gewächshaus wird unmittelbar nach der Einsaat der Versuchspflanzen in Saatschalen die Erdoberfläche mit einer wäßrigen Dispersion der Wirkstoffe, erhalten aus einem 25%igen Emulsionskonzentrat resp. aus einem 25%igen Spritzpulver mit Wirkstoffen, die wegen ungenügender Löslichkeit nicht als Emulsionskonzentrate hergestellt werden können, behandelt. Es wurden Konzentrationen von 4 kg Wirksubstanz/Hektar angewendet. Die Saatschalen werden im Gewächshaus bei 22–25°C und 50–70% relativer Luftfeuchtigkeit gehalten und der Versuch nach 3 Wochen ausgewertet und die Resultate nach folgender Notenskala bonitiert:

1 = Pflanzen nicht gekeimt oder total abgestorben
2–3 = sehr starke Wirkung
4–6 = mittlere Wirkung
7–8 = geringe Wirkung
9 = keine Wirkung (wie unbehandelte Kontrolle)

Pre-emergente Wirkung

Aufwandmenge: 4 kg Wirksubstanz/Hektar

| Verb. Nr. | Avena | Setaria | Sinapis | Stellaria |
|---|---|---|---|---|
| 3 | 2 | 3 | 1 | 2 |
| 5 | 1 | 2 | 1 | 2 |
| 7 | 2 | 2 | 1 | 1 |
| 8 | 4 | 4 | 1 | 1 |
| 13 | 3 | 3 | 1 | 2 |
| 18 | 2 | 3 | 1 | 1 |
| 22 | 1 | 1 | 1 | 1 |
| 39 | 1 | 1 | 1 | 1 |
| 40 | 1 | 2 | 1 | 1 |
| 101 | 3 | 3 | 9 | 2 |
| 102 | 2 | 1 | 9 | 1 |

Beispiel 8

Selektive pre-emergente Herbizidwirkung

Unter den gleichen Versuchsbedingungen wie in Beispiel 7 wurden eine größere Anzahl von Unkräuter- und Kulturpflanzensamen bei verschiedenen Wirkstoffkonzentrationen, entsprechend 4, 2, 1 und 0,5 kg/ha, geprüft. Die Bewertung erfolgte nach dem gleichen Maßstab.

| Testpflanze | Wirkung<br>Aufwandmenge<br>kg AS/ha<br><br>Verbindung Nr. 23 | | | |
|---|---|---|---|---|
| | 4 | 2 | 1 | 0,5 |
| Gerste | 9 | 9 | 9 | 9 |
| Weizen | 7 | 9 | 9 | 9 |
| Mais | 4 | 9 | 9 | 9 |
| Sorghum hybr. | 9 | 9 | 9 | 9 |
| Reis trocken | 2 | 4 | 7 | 9 |
| Avena fatua | 2 | 8 | 9 | 9 |
| Bromus tectorum | 2 | 3 | 3 | 9 |
| Lolium perenne | 5 | 8 | 9 | 9 |
| Alopecurus myos. | 2 | 5 | 7 | 9 |
| Digitaris sang. | 1 | 1 | 1 | 4 |
| Echinochloa s.g. | 2 | 8 | 9 | 9 |
| Sorghum halep. | 9 | 9 | 9 | 9 |
| Rottboellia ex. | 3 | 8 | 9 | 9 |
| Cyperus escul. | 5 | 9 | 9 | 9 |
| Soja | 3 | 9 | 9 | 9 |
| Baumwolle | 1 | 8 | 9 | 9 |
| Abutilon | 1 | 1 | 3 | 9 |
| Sida spinosa | 1 | 1 | 1 | 8 |
| Xanthium Sp. | 1 | 2 | 9 | 9 |
| Amaranthus ret. | 1 | 1 | 1 | 1 |
| Chenopodium Sp. | 1 | 1 | 1 | 1 |
| Solanum nigrum | 1 | 1 | 1 | 1 |
| Ipomoea | 2 | 7 | 9 | 9 |
| Sinapis | 1 | 1 | 1 | 4 |

Fortsetzung

| Testpflanze | Wirkung Aufwandmenge kg AS/ha Verbindung Nr. 23 | | | |
|---|---|---|---|---|
| | 4 | 2 | 1 | 0,5 |
| Stellaria | 1 | 1 | 1 | 2 |
| Chrysanthe. leuc. | 1 | 1 | 1 | 2 |
| Galium aparine | 1 | 3 | 8 | 9 |
| Viola tricolor | 1 | 1 | 2 | 4 |
| Veronica Sp. | 1 | 1 | 1 | 1 |
| Portulaca | – | – | – | – |
| Kochia scop. | – | – | – | – |

In diesem Versuch zeigten auch die übrigen Verbindungen der Formel I ausgezeichnete Herbizidwirkung gegen die meisten dikotylen und einige monokotyle Unkräuter, wobei die Kulturpflanzen wie Weizen, Gerste, Mais, Hirse, Soja und Baumwolle nicht oder erst in höheren Aufwandmengen geschädigt werden.

Beispiel 9

Pre-emergente Herbizid-Wirkung in Raps

Unter den gleichen Bedingungen wie im Beispiel 7 werden Rapspflanzensamen und einige typische Rapsunkräutersamen geprüft und ausgewertet.

Pre-emergente Wirkung

Verbindung Nr. 102

| Testpflanze | Wirkung | | |
|---|---|---|---|
| | 4 kg/ha | 2 kg/ha | 1 kg/ha |
| Raps ›Gulle‹ | 9 | 9 | 9 |
| Raps ›Bistro‹ | 9 | 9 | 9 |
| Alopecurus myos. | 4 | 6 | 8 |
| Matricaria cham. | 1 | 1 | 1 |
| Papaver rhoeas | 1 | 1 | 2 |
| Rumex crispus | 3 | 4 | 6 |
| Stellaria media | 1 | 4 | 5 |

Ein ähnliches Wirkungsspektrum zeigte die Verbindung Nr. 101.

Beispiel 10

Post-emergente Herbizid-Wirkung (Kontaktherbizid)

Eine Anzahl Unkräuter, sowohl monokotyle wie dikotyle, wurden nach dem Auflaufen (in 4- bis 6-Blattstadium) mit einer wäßrigen Wirkstoffdispersion in einer Dosierung von 4 kg Wirksubstanz pro Hektar auf die Pflanzen gespritzt und diese bei 24°–26°C und 45–60% relativer Luftfeuchtigkeit gehalten. Mindestens 15 Tage nach der Behandlung wird der Versuch ausgewertet und das Ergebnis nach derselben Notenskala wie im pre-emergenten Versuch (Beispiel 7) bonitiert.

Post-emergente Wirkung

Aufwandmenge: 4 kg Wirksubstanz/Hektar

| Verb. Nr. | Avena | Setaria | Lolium | Solanum | Sinapis | Stellaria |
|---|---|---|---|---|---|---|
| 1 | 5 | 4 | 7 | 1 | 1 | 1 |
| 2 | 1 | 1 | 1 | 1 | 1 | 1 |
| 3 | 1 | 2 | 3 | 1 | 1 | 1 |
| 4 | 2 | 2 | 5 | 1 | 1 | 1 |
| 5 | 2 | 3 | 4 | 1 | 1 | 1 |
| 6 | 2 | 1 | 2 | 1 | 1 | 1 |
| 7 | 3 | 1 | 2 | 1 | 1 | 1 |
| 8 | 4 | 2 | 5 | 1 | 1 | 1 |
| 11 | 3 | 1 | 2 | 1 | 1 | 1 |
| 12 | 2 | 1 | 3 | 1 | 1 | 1 |
| 14 | 4 | 2 | 5 | 1 | 1 | 1 |
| 15 | 5 | 4 | 5 | 1 | 1 | 1 |
| 19 | 1 | 1 | 5 | 1 | 1 | 1 |
| 22 | 2 | 2 | 2 | 1 | 1 | 1 |
| 23 | 1 | 1 | 3 | 1 | 1 | 1 |
| 29 | 3 | 1 | 6 | 1 | 1 | 1 |
| 34 | 1 | 3 | 3 | 1 | 1 | 1 |
| 38 | 5 | 5 | 6 | 1 | 1 | 1 |
| 39 | 2 | 2 | 2 | 1 | 1 | 1 |
| 40 | 2 | 1 | 2 | 1 | 1 | 1 |
| 41 | 6 | 5 | 6 | 1 | 1 | 1 |
| 42 | 4 | 6 | 4 | 1 | 1 | 1 |
| 44 | 4 | 4 | 3 | 1 | 1 | 1 |
| 45 | 6 | 4 | 5 | 1 | 1 | 2 |

Fortsetzung

| Verb. Nr. | Avena | Setaria | Lolium | Solanum | Sinapis | Stellaria |
|---|---|---|---|---|---|---|
| 46 | 5 | 5 | 4 | 1 | 1 | 1 |
| 47 | 4 | 4 | 4 | 1 | 1 | 1 |
| 48 | 4 | 4 | 4 | 1 | 1 | 1 |
| 114 | 7 | 3 | 6 | 1 | 1 | 1 |
| 119 | 5 | 7 | 6 | 1 | 1 | 1 |
| 120 | 5 | 3 | 5 | 1 | 1 | 1 |

Beispiel 11

Unter den gleichen Versuchsbedingungen wie im Beispiel 10 wurde eine größere Anzahl von Unkräutern und Kulturpflanzen bei verschiedenen Wirkstoffkonzentrationen, entsprechend 4, 2, 1 und 0,5 kg/ha oder 2, 1, 0,5 und 0,25 kg/ha geprüft. Die Bewertung erfolgte nach dem gleichen Maßstab.

| Testpflanze | Wirkung Aufwandmenge kg AS/ha | | | | | | | | | | | |
|---|---|---|---|---|---|---|---|---|---|---|---|---|
| | Verbindung Nr. 23 | | | | Verbindung Nr. 38 | | | | Verbindung Nr. 102 | | | |
| | 2 | 1 | 0,5 | 0,25 | 4 | 2 | 1 | 0,5 | 4 | 2 | 1 | 0,5 |
| Gerste | 3 | 8 | 9 | 9 | 9 | 9 | 9 | 9 | 4 | 7 | 9 | 9 |
| Weizen | 2 | 4 | 9 | 9 | 8 | 9 | 9 | 9 | 4 | 7 | 7 | 8 |
| Mais | 1 | 3 | 7 | 9 | 7 | 9 | 9 | 9 | 6 | 9 | 9 | 9 |
| Sorghum hybr. | 4 | 7 | 9 | 9 | 8 | 9 | 9 | 9 | 7 | 8 | 9 | 9 |
| Reis trocken | 2 | 3 | 7 | 9 | 7 | 7 | 9 | 9 | 2 | 4 | 7 | 9 |
| Avena fatua | 1 | 2 | 7 | 9 | 6 | 8 | 9 | 9 | 2 | 3 | 6 | 9 |
| Bromus tectorum | 1 | 2 | 2 | 7 | 6 | 9 | 9 | 9 | 7 | 7 | 8 | 9 |
| Lolium perenne | 3 | 3 | 9 | 9 | 9 | 9 | 9 | 9 | 4 | 6 | 9 | 9 |
| Alopecurus myos. | 2 | 2 | 8 | 9 | 3 | 6 | 9 | 9 | 2 | 3 | 3 | 8 |
| Digitaria sang. | 1 | 1 | 1 | 2 | 1 | 3 | 9 | 9 | 3 | 3 | 6 | 9 |
| Echinochloa c.g. | 1 | 1 | 2 | 7 | 7 | 9 | 9 | 9 | 7 | 9 | 9 | 9 |
| Sorghum halep. | 9 | 9 | 9 | 9 | 9 | 9 | 9 | 9 | 9 | 9 | 9 | 9 |
| Rottboellia ex. | 1 | 1 | 2 | 4 | 9 | 9 | 9 | 9 | 4 | 4 | 8 | 9 |
| Cyperus escul. | 4 | 4 | 7 | 9 | 4 | 6 | 9 | 9 | 7 | 7 | 9 | 9 |
| Soja | 1 | 1 | 1 | 2 | 3 | 6 | 8 | 9 | 1 | 2 | 2 | 3 |
| Baumwolle | 2 | 2 | 3 | 4 | 8 | 9 | 9 | 9 | 1 | 1 | 1 | 2 |

Fortsetzung

| Testpflanze | Wirkung Aufwandmenge kg AS/ha | | | | | | | | | | | |
|---|---|---|---|---|---|---|---|---|---|---|---|---|
| | Verbindung Nr. 23 | | | | Verbindung Nr. 38 | | | | Verbindung Nr. 102 | | | |
| | 2 | 1 | 0,5 | 0,25 | 4 | 2 | 1 | 0,5 | 4 | 2 | 1 | 0,5 |
| Abutilon | 1 | 1 | 1 | 1 | 2 | 2 | 3 | 3 | 1 | 1 | 1 | 1 |
| Sida spinosa | 1 | 1 | 2 | 3 | 4 | 6 | 8 | 9 | 2 | 6 | 7 | 7 |
| Xanthium Sp. | 1 | 1 | 1 | 1 | 1 | 1 | 2 | 2 | – | – | – | – |
| Amaranthus ret. | 1 | 1 | 1 | 1 | 1 | 2 | 5 | 6 | 1 | 1 | 4 | 4 |
| Chenopodium Sp. | 1 | 1 | 1 | 1 | 1 | 1 | 1 | 1 | – | – | – | – |
| Solanum nigrum | 1 | 1 | 1 | 1 | 1 | 1 | 1 | 1 | 1 | 1 | 1 | 1 |
| Ipomoea | 1 | 1 | 1 | 1 | 1 | 1 | 2 | 9 | 1 | 1 | 2 | 3 |
| Sinapis | 1 | 1 | 1 | 1 | 1 | 1 | 1 | 1 | 9 | 9 | 9 | 9 |
| Stellaria | 1 | 1 | 1 | 1 | 1 | 1 | 1 | 2 | 1 | 1 | 2 | 2 |
| Chrysanthe. leuc. | 1 | 1 | 1 | 1 | 1 | 1 | 1 | 1 | 1 | 1 | 3 | 4 |
| Galium aparine | 1 | 1 | 1 | 2 | 1 | 1 | 1 | 6 | 2 | 2 | 3 | 7 |
| Viola tricolor | 1 | 2 | 4 | 5 | 1 | 2 | 3 | 7 | – | – | – | – |
| Veronica Sp. | 1 | 1 | 1 | 1 | 1 | 1 | 6 | 7 | – | – | – | – |
| Portulaca | – | – | – | – | – | – | – | – | 1 | 1 | 1 | 1 |
| Kochia scop. | – | – | – | – | – | – | – | – | 1 | 1 | 1 | 2 |
| Sesbania | – | – | – | – | – | – | – | – | 1 | 1 | 1 | 1 |

Auch in diesem Versuch zeigten die Verbindungen der Formel I ausgezeichnete Wirkung gegen die meisten dikotylen und einige der monokotylen Unkräuter, wobei die Kulturpflanzen Soja, Mais, die Getreidearten Gerste, Hirse und Weizen wie auch Baumwolle nicht oder erst mit höheren Aufwandmengen Wirkstoff geschädigt wurden.

Neben guter kontakt-herbizider Wirkung zeigten einige Verbindungen der Formel I, insbesondere die Verbindungen Nr. 101 und 102, überraschend gute Selektivität in Rapskulturen.

**Patentansprüche**

1. Cyanoalkyl-phenylharnstoffe der Formel I

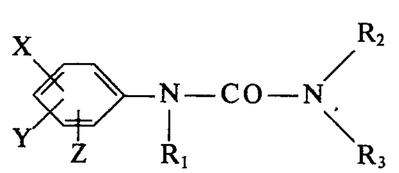

(I)

worin

X Wasserstoff, Halogen, Trifluormethyl, Methyl oder Methoxy,

Y eine Cyanoalkylgruppe 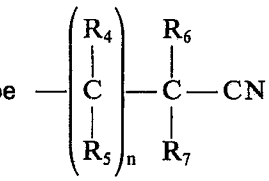

Z Wasserstoff oder Halogen,
n die Zahl 0, 1 oder 2,
$R_1$ Wasserstoff oder $C_1-C_6$-Alkyl,
$R_2$ Wasserstoff, $C_1-C_6$-Alkyl oder $C_1-C_6$-Alkoxy, ferner $C_2-C_6$-Alkenyl oder $C_3-C_6$-Alkinyl,
$R_3$ Wasserstoff, $C_1-C_6$-Alkyl oder $C_2-C_6$-Alkenyl oder
$R_2$ und $R_3$ zusammen mit dem Stickstoffatom, an das sie gebunden sind, einen 5–7gliedrigen Heterocyclus bilden, der auch ein Sauerstoff- oder Schwefelatom oder eine Iminogruppe als Ringglied enthalten kann,
$R_4$, $R_5$, $R_6$ Wasserstoff, $C_1-C_6$-Alkyl oder $C_1-C_6$-Halogenalkyl,
$R_7$ Wasserstoff, $C_1-C_6$-Alkyl, Aralkyl, insbesondere Benzyl, Phenyl oder $C_1-C_6$-Alkoxy oder
$R_6$ und $R_7$ zusammen mit dem sie tragenden Kohlenstoffatom einen $C_3-C_7$-Cycloalkylring bilden, bedeuten.

2. Verbindungen der Formel I gemäß Anspruch 1, dadurch gekennzeichnet, daß der Cyanoalkylsubstituent Y die para- oder meta-Position des Phenylkerns besetzt.
3. Verbindungen der Formel I gemäß Anspruch 1, dadurch gekennzeichnet, daß n für Null oder eins steht.
4. Verbindungen der Formel I gemäß Anspruch 1, dadurch gekennzeichnet, daß $R_2$ für Wasserstoff, Methyl oder Methoxy und $R_3$ für Methyl stehen.
5. Verbindungen der Formel I gemäß Anspruch 1, dadurch gekennzeichnet, daß $R_1$ Wasserstoff bedeutet.
6. Verbindungen der Formel I gemäß Anspruch 1, dadurch gekennzeichnet, daß Z Wasserstoff bedeutet.
7. Verbindungen der Formel I gemäß Anspruch 1, dadurch gekennzeichnet, daß X Wasserstoff, Halogen oder Trifluormethyl bedeutet.
8. Verbindungen der Formel I gemäß Ansprüchen 2 und 3, dadurch gekennzeichnet, daß der Cyanoalkylsubstituent Y die para-Position des Phenylrings besetzt und n Null bedeutet.
9. Verbindungen der Formel I gemäß Ansprüchen 2 und 3, dadurch gekennzeichnet, daß der Cyanoalkylsubstituent Y die meta-Position des Phenylrings besetzt und n eins bedeutet.
10. Verbindungen der Formel I gemäß Ansprüchen 4 bis 8, dadurch gekennzeichnet, daß die Substituenten Y in der para-Stellung und X in der meta-Stellung des Phenylkerns stehen, n Null ist, $R_1$ und Z Wasserstoff, $R_2$ Wasserstoff, Methyl oder Methoxy, $R_3$ Methyl und X Wasserstoff, Chlor oder Trifluormethyl bedeuten.
11. Verbindungen der Formel I gemäß Ansprüchen 4 bis 7 und 9, dadurch gekennzeichnet, daß der Substituent Y in der meta-Stellung des Phenylkerns steht, n eins ist, $R_1$, X und Z Wasserstoff, $R_2$ Wasserstoff, Methyl oder Methoxy und $R_3$ Methyl bedeuten.
12. Verbindungen der Formel I gemäß Anspruch 1, ausgewählt aus der Gruppe

β-[4-(3,3-Dimethylureido)-3-chlorphenyl]-propionitril,
β-[4-(3-Methoxy-3-methylureido)-3-chlorphenyl]-propionitril,
β-[3-(3-Methoxy-3-methylureido)-phenyl]-propionitril,
β-[3-(3,3-Dimethylureido)-phenyl]-propionitril,
α-[4-(3-Methoxy-3-methylureido)-3-chlorphenyl]-acetonitril,
α-[4-(3,3-Dimethylureido)-3-chlorphenyl]-acetonitril,
α-[4-(3,3-Dimethylureido)-3-trifluormethylphenyl]-acetonitril,
β-[4-(3,3-Dimethylureido)-3-methylphenyl]-α-methyl-propionitril.

13. Verfahren zur Herstellung der Cyanoalkyl-phenylharnstoffe der Formel I, dadurch gekennzeichnet, daß man in einem den Reaktionsteilnehmern gegenüber inerten organischen Lösungsmittel unter Verwendung der äquivalenten Menge einer Base ein Anilin der Formel II

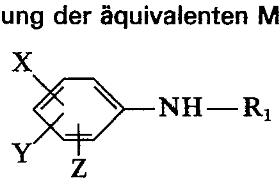

(II)

worin X, Y, Z und $R_1$ die unter Formel I, Anspruch 1, gegebene Bedeutung haben, mit einem Carbamoylchlorid der Formel III

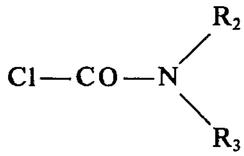

(III)

worin $R_2$ und $R_3$ die in Formel I, Anspruch 1, gegebene Bedeutung haben, umsetzt.

14. Verfahren zur Herstellung der neuen Cyanoalkyl-phenylharnstoffe der Formel I, dadurch gekennzeichnet, daß man in einem den Reaktionspartnern gegenüber inerten organischen Lösungsmittel ein Isocyanat der Formel IV

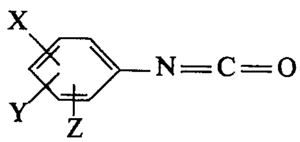

(IV)

worin X, Y und Z die unter Formel I, Anspruch 1, gegebene Bedeutung haben, mit einem Amin der Formel V

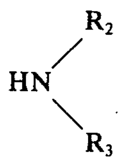

(V)

worin $R_2$ und $R_3$ die unter Formel I, Anspruch 1, gegebene Bedeutung haben, umsetzt.

15. Herbizides Mittel, dadurch gekennzeichnet, daß es neben Träger- und/oder anderen Zuschlagstoffen als Wirkstoff mindestens einen Cyanoalkyl-phenylharnstoff der Formel I, Anspruch 1, enthält.

16. Herbizides Mittel gemäß Anspruch 15, dadurch gekennzeichnet, daß es eine Verbindung gemäß einem der Ansprüche 2 bis 12 enthält.

17. Herstellung eines herbiziden Mittels gemäß einem der Ansprüche 15 oder 16, dadurch gekennzeichnet, daß man Träger- und/oder andere Zuschlagstoffe mit den Wirkstoffen der Formel I innig vermischt und vermahlt.

18. Die Verwendung der Cyanoalkyl-phenylharnstoffe der Formel I, Anspruch 1, oder sie enthaltender Mittel zur Bekämpfung von Unkräutern.

19. Die Verwendung gemäß Anspruch 18 zur selektiven Bekämpfung von Unkräutern in Raps.

20. Die Verwendung gemäß Anspruch 18 zur selektiven Bekämpfung von Unkräutern in Getreide.

**Claims**

1. A cyanoalkyl-phenylurea of the formula I

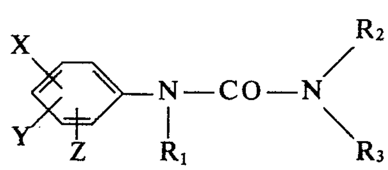

(I)

wherein

X is hydrogen, halogen, trifluoromethyl, methyl or methoxy,

Y is a cyanoalkyl group 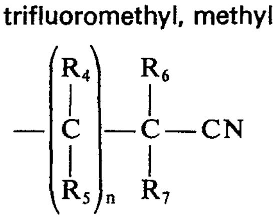

Z is hydrogen or halogen,
n is the number 0, 1 or 2,
$R_1$ is hydrogen or $C_1-C_6$-alkyl,
$R_2$ is hydrogen, $C_1-C_6$-alkyl, $C_1-C_6$-alkoxy, $C_2-C_6$-alkenyl or $C_3-C_6$-alkynyl,
$R_3$ is hydrogen, $C_1-C_6$-alkyl or $C_2-C_6$-alkenyl, or
$R_2$ and $R_3$ together with the nitrogen atom to which they are bound form a 5−7-membered heterocycle, which can contain as ring member also an oxygen or sulfur atom or an imino group,
$R_4$, $R_5$ and $R_6$ are each hydrogen, $C_1-C_6$-alkyl or $C_1-C_6$-haloalkyl,
$R_7$ is hydrogen, $C_1-C_6$-alkyl, aralkyl, particularly benzyl, phenyl or $C_1-C_6$-alkoxy, or
$R_6$ and $R_7$ together with the carbon atom carrying them can form a $C_3-C_7$-cycloalkyl ring.

2. A compound of the formula I according to Claim 1, wherein the cyanoalkyl substituent Y occupies the para- or meta-position of the phenyl ring.
3. A compound of the formula I according to Claim 1, wherein n is zero or one.
4. A compound of the formula I according to Claim 1, wherein $R_2$ is hydrogen, methyl or methoxy, and $R_3$ is methyl.
5. A compound of the formula I according to Claim 1, wherein $R_1$ is hydrogen.
6. A compound of the formula I according to Claim 1, wherein Z is hydrogen.
7. A compound of the formula I according to Claim 1, wherein X is hydrogen, halogen or trifluoromethyl.
8. A compound of the formula I according to Claims 2 and 3, wherein the cyanoalkyl substituent Y occupies the para-position of the phenyl ring, and n is zero.
9. A compound of the formula I according to Claims 2 and 3, wherein the cyanoalkyl substituent Y occupies the meta-position of the phenyl ring, and n is one.
10. A compound of the formula I according to any one of Claims 4 to 8, wherein the substituent Y is in the para-position and the substituent X in the meta-position of the phenyl ring, n is zero, $R_1$ and Z are each hydrogen, $R_2$ is hydrogen, methyl or methoxy, $R_3$ is methyl, and X is hydrogen, chlorine or trifluoromethyl.
11. A compound of the formula I according to any one of Claims 4 to 7 and 9, wherein the substituent Y is in the meta-position of the phenyl ring, n is one, $R_1$, X and Z are each hydrogen, $R_2$ is hydrogen, methyl or methoxy, and $R_3$ is methyl.
12. A compound of the formula I according to Claim 1 selected from the group comprising:

β-[4-(3,3-dimethylureido)-3-chlorophenyl]-propionitrile,
β-[4-(3-methoxy-3-methylureido)-3-chlorophenyl]-propionitrile,
β-[3-(3-methoxy-3-methylureido)-phenyl]-propionitrile,
β-[3-(3,3-dimethylureido)-phenyl]-propionitrile,
α-[4-(3-methoxy-3-methylureido)-3-chlorophenyl]-acetonitrile,
α-[4-(3,3-dimethylureido)-3-chlorophenyl]-acetonitrile,
α-[4-(3,3-dimethylureido)-3-trifluoromethylphenyl]-acetonitrile, and
β-[4-(3,3-dimethylureido)-3-methylphenyl]-α-methylpropionitrile.

13. A process for producing a cyanoalkyl-phenylurea of the formula I, Claim 1, which process comprises reacting, in an organic solvent inert to the reactants, with the use of the equivalent amount of a base, an aniline of the formula II

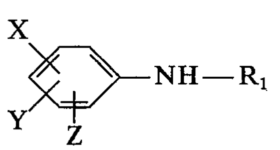

(II)

wherein X, Y, Z and $R_1$ have the meanings defined under the formula I, Claim 1, with a carbamoyl chloride of the formula III

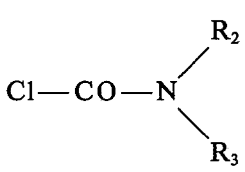

(III)

wherein $R_2$ and $R_3$ have the meanings defined under the formula I, Claim 1.
14. A process for producing a novel cyanoalkyl-phenylurea of the formula I, Claim 1, which process comprises reacting, in an organic solvent inert to the reactants, an isocyanate of the formula IV

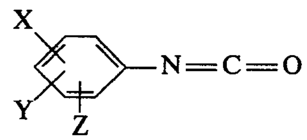

(IV)

wherein X, Y and Z have the meanings defined under the formula I, Claim 1, with an amine of the formula V

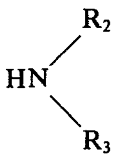

(V)

wherein $R_2$ and $R_3$ have the meanings defined under the formula I, Claim 1.

15. A herbicidal composition containing as active ingredient at least one cyanoalkyl-phenylurea of the formula I, Claim 1, together with carriers and/or other additives.

16. A herbicidal composition according to Claim 15, which composition contains a compound according to any one of Claims 2 to 12.

17. A process for producing a herbicidal composition according to Claim 15 or 16, which process comprises intimately mixing and grinding carriers and/or other additives with the active substance of the formula I, Claim 1.

18. A method of combating weeds at a locus, which method comprises applying to the locus a cyanoalkyl-phenylurea of the formula I, Claim 1, or a composition containing it.

19. A method according to Claim 18 for selectively combating weeds in rape crops.

20. A method according to Claim 18 for selectively combating weeds in cereal crops.

**Revendications**

1. Cyanoalkyl-phénylurées de formule I:

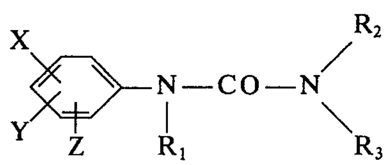

(I)

dans laquelle:

X représente de l'hydrogène, un halogène, un trifluorométhyle, un méthyle ou un éthoxy;

Y représente un groupe cyanoalkyle 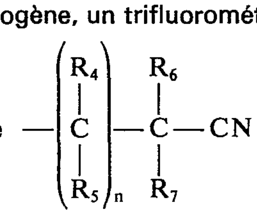

Z représente de l'hydrogène ou un halogène,
n représente le nombre 0, 1 ou 2,
$R_1$ représente de l'hydrogène ou un alkyle en $C_1$ à $C_6$,
$R_2$ représente de l'hydrogène, un alkyle en $C_1$ à $C_6$ ou un alcoxy en $C_1$ à $C_6$, ou encore un alcényle en $C_2$ à $C_6$ ou un alcynyleen $C_3$ à $C_6$,
$R_3$ représente de l'hydrogène, un alkyle en $C_1$ à $C_6$, ou un alcényle en $C_2$ à $C_6$ ou bien
$R_2$ et $R_3$ forment ensemble avec l'atome d'azote auquel ils sont reliés un hétérocycle de 5 à 7 chaînons, qui peut contenir également un atome d'oxygène ou un atome de soufre ou un groupe imino comme membre du cycle,
$R_4$, $R_5$, $R_6$ représentent de l'hydrogène, un alkyle en $C_1$ à $C_6$ ou un halogènoalkyle en $C_1$ à $C_6$,
$R_7$ représente de l'hydrogène, un alkyle en $C_1$ à $C_6$ un aralkyle, en particulier un benzyle ou un phényle ou un alcoxy en $C_1$ à $C_6$ ou bien
$R_6$ et $R_7$ forment ensemble avec l'atome de carbone qui les porte un noyau cycloalkyle en $C_3$ à $C_7$.

2. Composés de formule I selon la revendication 1, caractérisés en ce que le substituant cyanoalkyle Y occupe la position para ou méta du noyau phényle.

3. Composés de formule I selon la revendication 1, caractérisés en ce que n est égal à 0 ou 1.

4. Composés de formule I selon la revendication 1, caractérisés en ce que $R_2$ représente de l'hydrogène, un méthyle ou un éthoxy et $R_3$ représente un méthyle.

5. Composés de formule I selon la revendication 1, caractérisés en ce que $R_1$ représente un hydrogène.

6. Composés de formule I selon la revendication 1, caractérisés en ce que Z représente de l'hydrogène.

7. Composés de formule I selon la revendication 1, caractérisés en ce que X représente de l'hydrogène, un halogène ou un trifluorométhyle.

8. Composés de formule I selon l'une des revendications 2 et 3, caractérisés en ce que le substituant cyanoalkyle Y occupe la position para du noyau phényle et en ce que n est égal à 0.

9. Composés de formule I selon l'une des revendications 2 et 3, caractérisés en ce que le substituant cyanoalkyle Y occupe la position méta du noyau phényle et en ce que n est égal à 1.

10. Composés de formule I selon l'une des revendications 4 à 8, caractérisés en ce que les substituants Y occupent la position para et X la position méta du noyau phényle, n est égal à 0, $R_1$ et Z représentent de l'hydrogène, $R_2$ représente de l'hydrogène, un méthyle ou un éthoxy, $R_3$ représente un méthyle et X représente de l'hydrogène, du chlore ou un trifluorométhyle.

11. Composés de formule I selon l'une des revendications 4 à 7 et 9, caractérisés en ce que le substituant Y occupe la position méta du noyau phényle, n est égal à 1, $R_1$, X et Z représentent de l'hydrogène, $R_2$ représente de l'hydrogène, un méthyle ou un éthoxy et $R_3$ représente un méthyle.

12. Composés de formule I selon la revendication 1, choisis dans le groupe formé par:

$\beta$-[4-(3,3-diméthyluréido)-3-chlorophényl]-propionitrile,
$\beta$-[4-(3-méthoxy-3-méthyluréido)-3-chlorophényl]-propionitrile;
$\beta$-[3-(3-méthoxy-3-méthyluréido)-phényl]-propionitrile;
$\beta$-[3-(3,3-diméthyluréido)-phényl]-propionitrile;
$\alpha$-[4-(3-méthoxy-3-méthyluréido)-3-chlorophényl]-acétonitrile;
$\alpha$-[4-(3,3-diméthyluréido)-3-chlorophényl]-acétonitrile;
$\alpha$-[4-(3,3-diméthyluréido)-3-trifluorométhylphényl]-acétonitrile;
$\beta$-[4-(3,3-diméthyluréido)-3-méthylphényl]-$\alpha$-méthylpropionitrile.

13. Procédé de préparation de cyanoalkyle phénylurées de formule I, caractérisé en ce que l'on fait réagir, dans un solvant organique inerte vis-à-vis des partenaires de la réaction et en utilisant la quantité équivalente d'une base, une aniline de formule II:

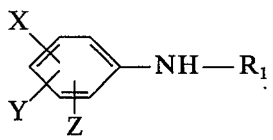

(II)

dans laquelle X, Y, Z et $R_1$ ont la signification donnée sous la formule I de la revendication 1, avec un chlorure de carbamoyle de formule III:

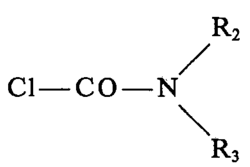

(III)

dans laquelle $R_2$ et $R_3$ ont la signification donnée pour la formule I de la revendication 1.

14. Procédé de préparation de nouvelles cyanoalkylphénylurées de formule I, caractérisé en ce que l'on fait réagir, dans un solvant organique inerte vis-à-vis des partenaires de la réaction, un isocyanate de formule IV:

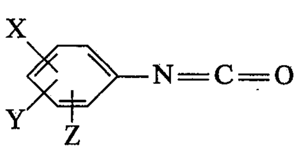

(IV)

dans laquelle X, Y et Z ont la signification donnée sous la formule I de la revendication 1, avec une amine

de formule V:

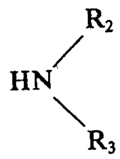

(V)

dans laquelle $R_2$ et $R_3$ ont la signification donnée sous la formule I de la revendication 1.

15. Agent herbicide, caractérisé en ce qu'il contient, outre des supports et/ou substances additionnelles, en tant que substance active au moins une cyanoalkylphénylurée de formule I selon la revendication 1.

16. Agent herbicide selon la revendication 15, caractérisé en ce qu'il contient un composé selon l'une des revendications 2 à 12.

17. Procédé de préparation d'un agent herbicide selon l'une des revendications 15 ou 16, caractérisé en ce que l'on mélange intimement et on broie des supports et/ou autres substances additionnelles avec les substances actives de formule I.

18. Utilisation des cyanoalkylephénylurées de formule I selon la revendication 1, ou d'agents les contenant pour la lutte contre les mauvaises herbes.

19. Utilisation selon la revendication 18, pour la lutte sélective des mauvaises herbes dans le colza.

20. Utilisation selon la revendication 18, pour la lutte sélective des mauvaises herbes dans les céréales.